# EUROPEAN PATENT SPECIFICATION

(11) **EP 1 761 272 B1**
(45) Date of publication and mention of the grant of the patent: **24.04.2013**
(21) Application number: 05752096.7
(22) Date of filing: 07.06.2005
(51) Int. Cl.: A61K 38/05, A61P 39/06

(54) **COMPOSITIONS CONTAINING D-CARNOSINE**
D-CARNOSIN ENTHALTENDE ZUSAMMENSETZUNGEN
COMPOSITIONS COMPRENANT DE LA D-CARNOSINE

(30) Priority: 08.06.2004 EP 04425421
(43) Date of publication of application: 14.03.2007
(73) Proprietor: Flamma S.P.A., 24040 Chignolo D'Isola (BG) (IT)
(72) Inventor: ALDINI, Giancarlo, I - 24040 CHIGNOLO D'ISOLA (BG) (IT); CANEVOTTI, Renato, I-24040 Chignolo D'Isola (IT); NEGRISOLI, Gianpaolo, I-24040 Chignolo D'Isola (IT)
(74) Representative: Minoja, Fabrizio
(86) International application number: PCT/EP2005/006090
(87) International publication number: WO 2005/120543

(56) References cited:
- EP-A- 1 285 654
- WO-A-92/09298
- US-A- 5 561 110
- US-A1- 2004 081 681
- DATABASE WPI Section Ch, Week 200301 Derwent Publications Ltd., London, GB; Class B03, AN 2003-010772 XP002304780 & KR 2002 044 740 A (KANG K S) 19 June 2002 (2002-06-19)
- ALDINI GIANCARLO ET AL: "Carnosine is a quencher of 4-hydroxy-nonenal: Through what mechanism of reaction?" BIOCHEMICAL AND BIOPHYSICAL RESEARCH COMMUNICATIONS, vol. 298, no. 5, 15 November 2002 (2002-11-15), pages 699-706, XP002304779 ISSN: 0006-291X cited in the application

## Description

The present invention relates to compositions containing D-carnosine as the active ingredient.

The invention also relates to the use of D-carnosine for the preparation of a medicament or diet supplement for the treatment or prevention of oxidative stress-induced disorders, especially disorders in which lipid peroxidation plays an important part.

### Background to the invention

L-carnosine is an endogenous dipeptide (β-alanyl-L-histidine) present in concentrations of up to 20 mM in the human muscles and nerve tissue.

A number of possible physiological functions of carnosine have been suggested in the past, one of the most widely studied being its ability to react with toxic aldehydes deriving from metabolic pathways of oxidative degradation of endogenous compounds such as carbohydrates, polyunsaturated fatty acids and proteins. L-carnosine thus appears to perform a protective and detoxifying effect on the toxic metabolites which are presumably responsible, or in any event jointly responsible, for a number of disorders.

One of these toxic metabolites which has recently attracted researchers' attention is 4-hydroxy-*trans*-2-nonenal or HNE, an alpha, beta-unsaturated aldehyde deriving from lipid peroxidation of unsaturated fatty acids such as arachidonic acid and linoleic acid.

HNE is highly reactive and forms adducts with different biomacromolecules, reacting, for example, with cysteine, lysine and histidine residues of proteins whose activity can thus be altered (Liu et al., Molecular Aspects of Medicine 24, 2003, 305-313). The same authors postulate that carbonylation of proteins plays an important part in the development of Alzheimer's disease.

A review by Uchida (Progress in Lipid Research, 42, 2003, 318-343) also discusses the pathogenetic role of HNE in various neurodegenerative disorders, such as Alzheimer's disease, Parkinson's disease and Amyotrophic Lateral Sclerosis (ALS), atherosclerosis and other disorders such as diabetic nephropathy, systemic amyloidosis, cancer, pre-eclampsia, primary biliary cirrhosis and vascular disorders. Finally, the role of HNE in the pathogenesis of neurodegenerative disorders is also discussed by Zarkovic K. in Molecular Aspects of Medicine 24, 2003, 293-303.

The mechanism whereby carnosine is able to detoxify cytotoxic alpha, beta-unsaturated aldehydes was studied by Aldini G. et al. in Biochem. Byophys. Res. Comm., 298,2002,699-706.

Acrolein, like HNE, is a carbonyl toxin deriving from lipid peroxidation which is present in significantly higher levels in the brains of patients suffering from Alzheimer's disease than in those of healthy subjects.

Carini et al. have characterised the adducts of carnosine and homocarnosine with acrolein using a particular mass spectrometry technique (J. Mass Spectrom., 38, 2003, 996-1006).

L-carnosine is known to be rapidly hydrolysed in human serum by specific hydrolytic enzymes (carnosinases), which obviously limits its possible therapeutic uses in the above-mentioned disorders (Pegova A., et al. Comp. Biochem. Physiol. Biochem. Mol. Bio. 127 (4), 2000: 443-446).

US 5561110 discloses the use of carnosine and related peptides to treat the complications of diabetes. D-carnosine is cited as equivalent to carnosine and other peptides such as anserine, ophidine, homocarnosine and homoanserine, although L-carnosine is clearly indicated as preferred. Example 4 of US 5561110 demonstrates that D-carnosine reacts with dihydroxyacetone at a substantially similar rate to L-carnosine.

Cosmetic uses of L-carnosine have also been disclosed (EP 1388339), in ophthalmology (WO 2004/028536), cardiology (US 5585396), as an anti-tumoral agent (DE 3424781), an energy-giving and anti-muscle fatigue agent (EP 449787), and a nutritional agent (EP 825871).

US 20047081681 discloses cosmetic formulations based on antioxidants: D-carnosine is cited together with L- and D,L- carnosine in a very long list of active ingredients. No actual exemplification or data concening D-carnosine is however given in this document.

WO 92/09298 discloses methods for delaying, preventing and/or reversing senescence of cells by means of carnosine-like compounds. Again, D-carnosine is generically cited together with anserine, ophidine, homocarnosine, homoanserine, carcinine etc. but the only evidence available concerns the natural occurring carnosine, i.e. the L-isomer.

KR 2002 044740 ( Database WPI Section Ch, AN 2003-010772) discloses an apoptosis suppressing agent comprising carnosine. Even though, once again, D-carnosine is just mentioned in the Abstract, the data obtained in the application refer only to the L-carnosine which is in fact explicitly recognised as the preferred agent in the description since "L-carnosine does not require a specific enzymatic function" (English translation of the third paragraph of the Section [Structure and Operation of the Invention]. This document therefore teaches away from using D-carnosine for the desired therapeutic purposes.

In conclusion, the prior art as a whole provides an enabling disclosure for the use of natural carnosine only, namely the L- form. Only US 556110 reports the reactivity of D- and L-carnosine towards carbonyl compounds as being substantially equivalent, without suggesting anyhow any advantageous effect of the D form in clinical applications.

### Description of the invention

It has now surprisingly been found that due to its favourable pharmacokinetic properties, D-carnosine produces better therapeutic results than the L- form considered to date.

D-carnosine is resistant to hydrolysis by the carnosinases, and is therefore stable in the plasma as well as being able to cross the blood-brain barrier.

The almost identical reactivity compared with the L- form towards cytotoxic aldehydes, especially HNE and acrolein, was confirmed in a homogenous solution of HNE (10 µm) and acrolein (200 µm) in 10 mM phosphate buffer in molar ratios of the dipeptides amounting to 1:10 and 1:20. After incubation for 1 and 14 h, aldehyde consumption was determined by conventional liquid chromatography techniques.

L- and D- carnosine inhibited HNE to the extent of 44 and 49.2% respectively after one hour's incubation, and 96.4 and 98.3% after 24 hours. These differences are not statistically significant, whereas D-carnosine is far more active towards HNE than aminoguanidine and methoxyamine, which are considered the most reactive compounds towards aldehydes. The reaction kinetics of D-carnosine with HNE follow a first-order profile.

The stability of L- and D-carnosine was compared in human plasma and serum *in vitro*, and *in vivo* in the rat (acute and chronic treatment). An LC-MS/MS technique, validated in the 5-250 µM range, with detection limits of 50-20 picomoles, was set-up for this purpose.

In the *in vitro* studies, D-carnosine remained unchanged after 6 hours incubation at 37°C in plasma obtained from human volunteers, in the presence and absence of cadmium chloride, a strong carnosinase activator. After 24 hours incubation, the D-carnosine concentration only fell by 6%. Conversely, L-carnosine was already undetectable after only one hour incubation under the same conditions. The results did not change substantially in the presence of cadmium ions. Similar results were obtained by using human serum, since already after 1 hour of incubation, L-carnosine was not detectable, while D-carnosine was found unchanged until 6 hours to slightly reduce by 7 % after 24 hours of incubation

In the *in vivo* acute studies, D- and L-carnosine were administered orally to Wistar rats (320 g ± 25 g) in the form of an aqueous solution at pH 7.4 (HCl) at the dose of 570 mg/kg.

The Cmax of L-carnosine was 60 minutes to drastically reduce and disappear within 2 hours form the administration. The pharamacokinetic profile of D-carnosine is completely different since the Cmax was reached at 2 hours, to maintain pharmacological levels up to 8 hours and disappear after 24 hours the administration. The AUC values for D-carnosine (94.98 ± 8.9) versus L-Carnosine (20.94 ± 1.97) unequivocally demonstrate a greater metabolic stability and bioavailability of D-carnosine.

In chronic treatment, D- and L-carnosine were administered with the diet water for 12 weeks at a dose of 30 mg/Kg. The experiment was carried out by using obese-Zucker rats (Charles River), an animal model of metabolic disorders, characterized by hyperinsulinemia, insulin resistance, obesity, hypertriglyceridemia, hypercholesterolemia, and by an increased formation of lipid derived carbonyls, protein carbonylation and appearance of carbonyl-dependent degenerative process such as nephropathy and vascular dysfunction, (Metz TO, Alderson NL, Chachich ME, Thorpe SR, Baynes JW, Pyridoxamine traps intermediates in lipid peroxidation reactions in vivo: evidence on the role of lipids in chemical modification of protein and development of diabetic complications, J Biol Chem. 2003 Oct 24;278(43):42012-9. Epub 2003 Aug 15).
The results indicate a 6 fold increase of D-carnosine (1.24 ± 0.3 nmoles/ml) in respect to L-carnosine (0.23 ± 0.2 nmoles/ml) in plasma and more than a ten-fold increase in urine (D-carnosine: 6688 ± 345 nmoles over 24 hrs; L-carnosine: 577 ± 121 nmoles over 24 hrs) as well as in the kidney (D-carnosine: 340.2 ± 12.3 nmoles/gr wt tissue; L-carnosine: 39.6 ± 4.1 nmoles/gr wt tissue). The data well demonstrate the metabolic stability of D-carnosine in respect to L-carnosine, leading to reach pharmacological concentration in plasma and in the kidney were it is excreted as unmodified form. Furthermore, these data clearly indicate that D-carnosine, maintaining the trapping activity and bioavailability of the parent compound and resulting more resistant to the enzymatic hydrolysis of carnosinases, can act as detoxifying agent towards those carbonyl compounds acting as cytotoxic intermediates in blood and kidney, responsible of vascular dysfunction as well as nephropathy.

Based on these results and considering the metabolic stability of D-carnosine but not of L-carnosine, in a second step it was studied the pharmacological effects of a chronic treatment of D-carnosine in Zucker rats. The results of this study unequivocally demonstrate the ability of D-carnosine to restrain: hypertriglyceridemia, hypercholesterolemia, hypertension, and carbonyl related disesases such as vascular dysfunction and nephropathy. D-carnosine, which combines high, rapid reactivity towards cytotoxic aldehydes (much greater than that of the drugs currently at clinical phase I) with total resistance to plasma carnosinases, can therefore be successfully used in the treatment of oxidative stress disorders. In particular, D-carnosine is effective for the treatment or prevention of disorders involving highly reactive and cytotoxic carbonyl derivatives (HNE, acrolein, malonyldialdehyde, glyoxal and dimethylglyoxal), especially nephropathy, vascular dysfunction, hypertriglyceridemia, hypercholesterolemia, hypertension in metabolic syndromes, sarcopenia. Forms designed for topical administration are particularly indicated for the latter.

For the uses considered, D-carnosine is conveniently formulated in conventional pharmaceutical, cosmetic or nutritional compositions suitable for oral, parenteral, topical or transdermal administration. Examples of these compositions include capsules, tablets, syrups, injectable solutions or suspensions, ointments, controlled-release forms and the like, drinks and diet bars. These forms, together with conventional vehicles and excipients, could also contain other active ingredients which have a complementary activity or are otherwise useful for the treatment/prevention of the disorders in question.

The daily dose will depend on a number of factors, such as the severity of the disorder to be treated and the patient's weight, sex and age, and can easily be determined by an expert doctor. Broadly speaking, however, in view of the substantial lack of toxicity of D-carnosine, the doses can vary within a wide range, for example from 100 mg to several grams a day (5 g or more a day), possibly divided into a number of daily administrations.

## Claims

1. Use of D-carnosine for the preparation of a medicament or diet supplement for the treatment of disorders induced by oxidative stress, wherein the disorder is sarcopenia.

2. Use of D-carnosine for the preparation of a medicament or diet supplement for the treatment of disorders induced by oxidative stress, wherein the disorder is selected from nephropathy, vascular dysfunction, hypertriglyceridemia, hypercholesterolemia and hypertension in metabolic syndromes.

## Patentansprüche

1. Verwendung von D-Carnosin für die Herstellung eines Medikaments oder eines Nahrungsergänzungsmittels für die Behandlung von Funktionsstörungen, die durch oxidativen Stress induziert werden, wobei die Funktionsstörung Sarkopenie ist.

2. Verwendung von D-Carnosin für die Herstellung eines Medikaments oder eines Nahrungsergänzungsmittels für die Behandlung von Funktionsstörungen, die durch oxidativen Stress induziert werden, wobei die Funktionsstörung ausgewählt ist aus Nephropathie, vaskularer Dysfunktion, Hypertriglyceridämie, Hypercholesterinämie und Hypertonie bei metabolischen Syndromen.

## Revendications

1. Utilisation de la D-carnosine pour la préparation d'un médicament ou d'un complément alimentaire pour le traitement de troubles induits par le stress oxydatif, dans laquelle le trouble est la sarcopénie.

2. Utilisation de la D-carnosine pour la préparation d'un médicament ou d'un complément alimentaire pour le traitement de troubles induits par le stress oxydatif, dans laquelle le trouble est choisi parmi une néphropathie, un dysfonctionnement vasculaire, une hypertriglycéridémie, une hypercholestérolémie et une hypertension dans des syndromes métaboliques.
